Europäisches Patentamt

European Patent Office

Office européen des brevets

(11) Veröffentlichungsnummer: **0 099 010 B1**

(12) **EUROPÄISCHE PATENTSCHRIFT**

(45) Veröffentlichungstag der Patentschrift:
10.04.85

(21) Anmeldenummer: 83106181.7

(22) Anmeldetag: 24.06.83

(51) Int. Cl.⁴: **A 61 F 5/30, A 63 B 71/12**

(54) **Stossneutralisator.**

(30) Priorität: 10.07.82 **DE 8219790 U**
17.02.83 **DE 3305408**

(43) Veröffentlichungstag der Anmeldung:
**25.01.84 Patentblatt 84/4**

(45) Bekanntmachung des Hinweises auf die Patenterteilung:
**10.04.85 Patentblatt 85/15**

(84) Benannte Vertragsstaaten:
**AT BE CH DE FR GB IT LI NL SE**

(56) Entgegenhaltungen:
**DE - C - 836 552**
**DE - U - 1 902 645**
**DE - U - 7 715 810**
**DE - U - 8 219 790**
**FR - A - 998 525**
**GB - A - 1 002 955**
**US - A - 2 889 830**
**US - A - 2 953 130**
**US - A - 3 526 221**

**Die Akte enthält technische Angaben, die nach dem Eingang der Anmeldung eingereicht wurden und die nicht in dieser Patentschrift enthalten sind.**

(73) Patentinhaber: **Wortberg, Walter, Dr. med., Buschhauser Weg 13b, D-5880 Lüdenscheid (DE)**

(72) Erfinder: **Wortberg, Walter, Dr. med., Buschhauser Weg 13b, D-5880 Lüdenscheid (DE)**

(74) Vertreter: **Hassler, Werner, Dr., Postfach 17 04 Asenberg 62, D-5880 Lüdenscheid (DE)**

## Beschreibung

Die Erfindung betrifft einen Stossneutralisator zum Schutz des menschlichen Körpers mit einem Querschnitt, dessen äussere Begrenzungslinie auf der dem menschlichen Körper abgewandten Seite glockenförmig verläuft, und mit einem ovalförmigen Umriss sowie einem mehrschichtigen Aufbau, wobei die an der Haut anliegende Unterseite eine hautfreundliche Haftklebeschicht aufweist.

Anwendungsgebiet der Erfindung ist der Schutz gefährdeter Knochen und Gelenke. Bei älteren Personen ist der Hüftbereich besonders gefährdet. Anwendungsgebiet der Erfindung ist auch der Schutz innerer Organe.

Bei älteren Personen ist der hüftgelenksnahe Oberschenkelbruch der häufigste Knochenbruch. Untersuchungen zeigen deutlich, dass die Zahl dieser hüftgelenksnahen Oberschenkelbrüche mit dem Alter der Personen zunimmt. Per-Axel Alffram, in „An Epidemiologic Study of cervical and trochanteric fractures of the femur in an urban population", „Acta orthop. scand." Supplementum Nr. 65, 1964 Malmö, konnte eindeutig nachweisen, dass der leichte Fall die häufigste Ursache für einen solchen Oberschenkelbruch ist. Eigene Untersuchungen haben dieses bestätigt.

Als Folge eines solchen hüftgelenksnahen Oberschenkelbruchs ergeben sich für die betroffenen Personen lange Krankenhausaufenthalte und eine lange Krankheitsdauer. Die Mortalität ist vergleichsweise hoch. In der Bundesrepublik Deutschland beträgt die Zahl solcher Oberschenkelbrüche bei älteren Menschen etwa 40 000 pro Jahr. Oberschenkelbrüche dieser Art stellen nicht nur ein präventivmedizinisches, sondern auch ein sozialmedizinisches Problem dar.

Für orthopädische Zwecke sind Druckpolster oder Pelotten aus Schaum- oder Moosgummi bekannt, vgl. DE-U Nrn. 1902645 und 7715810. Diese Druckpolster sollen einen Druck oder eine Stützkraft auf bestimmte Hautpartien oder Gewerbepartien ausüben. Als Stossabsorber, der möglichst viel Stossenergie oder Fallenergie absorbieren soll, ist ein solches Druckpolster nicht geeignet.

Die US-A Nr. 2889830 beschreibt einen Stossneutralisator, der mit einem Hüftgürtel und mit Schenkelbändern getragen wird. Hierdurch ergeben sich Probleme für eine sichere Ausrichtung des Stossneutralisators auf den zu schützenden Körperteil. Ausserdem ist die Anordnung einer Schaumgummischicht in der Nachbarschaft der Haut und einer Dämpfungsfeder an der Aussenseite kinematisch ungünstig. Denn es kann bei Stössen und ähnliche Belastungen zu einem Durchdrücken der Schaumgummischicht kommen, so dass dann der Stossneutralisator unwirksam wird. Entsprechendes gilt für den Stossneutralisator nach der US-A Nr. 3526221.

Das DE-U Nr. 8219790 beschreibt einen Stossneutralisator der eingangs genannten Art. Dort wird ein nachgiebiges Kissen beschrieben, das mittels einer Klebstoffschicht auf der Haut getragen wird. Das nachgiebige Kissen bestimmt die Dämpfungs- und Absorptionswirkung.

Aufgabe der Erfindung ist die Bereitstellung eines Stossneutralisators mit wesentlich verbesserter Absorptionswirkung.

Diese Aufgabe wird nach der Erfindung dadurch gelöst, dass eine an der Oberseite liegende Aussenschicht aus einem gummielastischen Stoff besteht und dass innerhalb der schalenförmigen Aussenschicht eine zähflüssige Fluidschicht liegt, die durch chemische Bindungen in der Grenzschicht mit der Aussenschicht integral verbunden ist. Die Aussenschicht kann dabei aus einem vernetzten Silikonkautschuk und die Fluidschicht aus einem untervernetzten Silikonkautschuk bestehen. Weitere Weiterbildungen der Erfindung sind in den Ansprüchen 3 bis 7 gekennzeichnet.

Der vernetzte Silikonkautschuk hat gummielastische Eigenschaften, der untervernetzte Silikonkautschuk fluidähnliche Eigenschaften. Die erfinderische Tätigkeit ist darin zu sehen, dass die zähflüssige Fluidschicht durch chemische Bindungen in der Grenzschicht mit der Aussenschicht integral verbunden ist. Die als Klebstoffschicht wirksame Fluidschicht sichert einen rutschfreien Sitz auf der Haut und trägt zur Dämpfung von Belastungen bei. Es zeigt sich, dass die Steifigkeit des Silikonkautschuks kraftabhängig ist. Durchgeführte Untersuchungen haben ergeben, dass die bei einem Fall auf den Trochanter major wirksame Stosskraft durch den Stossneutralisator auf etwa 30 bis 45% derjenigen Stosskraft, die ohne Stossneutralisator wirksam ist, vermindert werden kann. Die Stosskraft wird also soweit herabgesetzt, das beim Fall normalerweise kein Bruch im hüftgelenksnahen Oberschenkelbereich auftritt. Da das Fluid der Fluidschicht inkompressibel ist, weicht bei einem Stoss die Fluidschicht aus und verdrängt die gummielastische Schicht. Durch diese Ausbildung erreicht man, dass die Fluidschicht selbst einen Grossteil der Fallenergie aufnimmt und in die Aussenschicht ableitet. Dies führt bei der elastischen Rückstellung zu einer verzögerten Freigabe der Stossenergie. Dadurch ergibt sich eine hohe Dämpfungswirkung.

Der Stossneutralisator für den hüftgelenksnahen Oberschenkelbereich kann auch als Hüftpelotte bezeichnet werden. Bei dieser Anwendung hat der Stossneutralisator einen ovalförmigen Umriss. Er wird mit seiner Grundfläche etwa in Längsrichtung des Oberschenkelknochens auf die Haut aufgeklebt, wobei der Bereich grösster Dicke den Trochanter major oder grossen Rollhügel überdeckt. Der Stossneutralisator ist im Bereich der grössten Dicke etwa 20 bis 50 mm, vorzugsweise 20 bis 40 mm dick. Die Ausdehnung des Trochanter major beträgt 6 bis 10 cm. Die Grundfläche des Stossneutralisators beträgt vorzugsweise 16 cm in der Breite und 20 cm in Längsrichtung des Oberschenkelknochens. Die Dicke der Haftklebeschicht beträgt einige Millimeter bis 40 mm.

Ferner schlägt die Erfindung vor, dass die Fluidschicht an der Unterseite des Stossneutralisators freiliegt und die Haftklebeschicht bildet. Somit wird die Fluidschicht auch als Haftklebeschicht ausgenutzt. Die Haftklebeschicht ist durch ein Schutzblatt abgedeckt.

Der vernetzte Silikonkautschuk ist weich und nachgiebig. Seine Härte lässt sich durch den bei der Verarbeitung zugesetzten Katalysatoranteil innerhalb eines weiten Bereichs entsprechend dem jeweiligen Anwendungszweck einstellen. Der Silikonkautschuk lässt sich leicht in jede Form verarbeiten. Er ist weitgehend unempfindlich gegenüber Temperaturänderungen, gegenüber Waschmitteln und gegen Abrieb. Es treten keine Materialermüdungen auf. Der Silikonkautschuk zeigt keine Feuchtigkeitsaufnahme. Er ist flexibel und reissfest. Ausserdem hat er gute Isolationseigenschaften gegen Kälte und Wärme. Dermatologisch ist der Silikonkautschuk unbedenklich. Weder sind Allergien bekannt, noch treten Ekzeme oder Scheuerstellen auf. Der Silikonkautschuk ist hautfreundlich, er scheuert nicht und passt sich schnell an die Körpertemperatur an.

Infolge des Haftklebeeffekts der Fluidschicht haftet der Stossneutralisator unmittelbar auf der Haut fest, so dass er dauernd, auch nachts, getragen werden kann. Damit ist auch ein Schutz gegen Fallen aus dem Bett gegeben, was bei älteren Personen wichtig ist. Weil ein Abnehmen des Stossneutralisators nicht notwendig ist, kann derselbe auch bei hygienischen und sanitären Verrichtungen getragen werden. Er bietet auch einen Schutz gegen Fallverletzungen bei der Bad- und Toilettenbenutzung. Die Haftklebeschicht stellt auch eine Fixierung des Stossneutralisators auf der Haut sicher, so dass sich derselbe nicht verschieben kann. Dies trägt zusätzlich zur Stossdämpfung bei.

Der fluidähnliche Silikonkautschuk der Selbstklebeschicht schmiegt sich dem Profil der Haut an und füllt insbesondere auch die Poren der Haut aus, so dass dadurch eine ausserordentlich feste Haftung des Stossneutralisators gewährleistet ist. Dies ist für die Dämpfungswirkung desselben gegenüber Fallbelastungen sehr wichtig.

In Weiterbildung der Erfindung ist vorgesehen, dass die Fluidschicht am Umfang Treppenstufen mit Hinterschneidungen aufweist. Dadurch erhält man eine besonders günstige Dämpfungsstruktur. Die Treppenstufen haben eine Wirkung von Tellerfedern.

Ferner sieht die Erfindung vor, dass der Stossneutralisator eine oder mehrere porenartige Luftkammern enthält. Hierdurch werden die Dämpfungseigenschaften in günstiger Weise beeinflusst.

In Weiterbildung der Erfindung ist vorgesehen, dass zur Verwendung im Hüftbereich die Unterseite des Stossneutralisators im Bereich des Trochanter major eine kalottenförmige Ausnehmung aufweist. Dieses erleichtert eine Ausrichtung und Fixierung des Stossneutralisators beim Anlegen an den Körper des Trägers.

Zur Anpassung an die weibliche und männliche Anatomie sowie an verschiedene Körpergrössen kann der Stossneutralisator in verschiedenen Grössen bereitgehalten werden. Es ist davon auszugehen, dass einige wenige Grössen, etwa vier Grössen, ausreichen. Auch eine Anwendung als Implantat ist möglich.

Weitere Anwendungen des Stossneutralisators sind der Schutz des Unterarms. Unterarmbrüche, insbesondere Radiusfrakturen im handgelenksnahen Bereich stehen an zweiter Stelle der Knochenbrüche bei älteren Menschen, bei Kindern — infolge Sportunfällen beim Schlittschulaufen, Rollschuhlaufen, Skateboardfahren u. dgl. — sogar an erster Stelle. Der Stossneutralisator wird auf die Handfläche oder nur auf Daumen und Kleinfingerballen aufgeklebt. Damit lässt sich der Fall auf die Hand, der meist Ursache einer Radiusfraktur ist, dämpfen.

Der Stossneutralisator lässt sich auch als Steissbeinpolster einsetzen. Dadurch erhält man einen Schutz gegen Kompressionsfrakturen der Wirbelsäule. Der Stossneutralisator kann hufeisenförmig ausgebildet sein, wobei die beiden Schenkel des Hufeisens die Analfalte umfassen.

Ferner eignet sich der Stossneutralisator als Kopfpolster zum Schutz von Stirn, Schläfenlappen und Hinterhaupt. Das Schädeldach kann durch entsprechende Querstege geschützt werden. Ein solcher Stossneutralisator ist für epilepsiekranke Kleinkinder, aber auch für grössere Kinder und Erwachsene geeignet, und auch als Schutz für Sportler.

Der Stossneutralisator eignet sich ferner als Unterschenkelpolster zum Schutz der Schienbeinkante. Der Stossneutralisator bedeckt die gesamte Schienbeinkante. Er eignet sich für Fussballspieler und Eishockeyspieler.

Der Stossneutralisator eignet sich auch zum Schutz innerer Organe, etwa als Schutzgürtel für Leber, Milz und Nieren für Motorradfahrer.

Ausführungsformen der Erfindung sind im folgenden unter Bezugnahme auf die anliegenden Zeichnungen erläutert, in denen darstellen:

Fig. 1 eine Vorderansicht des Beckenbereiches mit einer Darstellung der Anordnung des Stossneutralisators,

Fig. 2 eine Seitenansicht der linken Körperhälfte,

Fig. 3 eine abgewandelte Ausführungsform eines Stossneutralisators in der Ansicht,

Fig. 4 einen Schnitt zu Fig. 3,

Fig. 5 eine Versuchsanordnung für Belastungsversuche,

Fig. 6 eine Erläuterung der Modellrechnungen an den Femurknochen,

Fig. 7 eine abgewandelte Ausführungsform eines Stossneutralisators, und

Fig. 8 einen Schnitt zu Fig. 7.

Es sind im folgenden Stossneutralisatoren für den Hüftgelenkbereich erläutert.

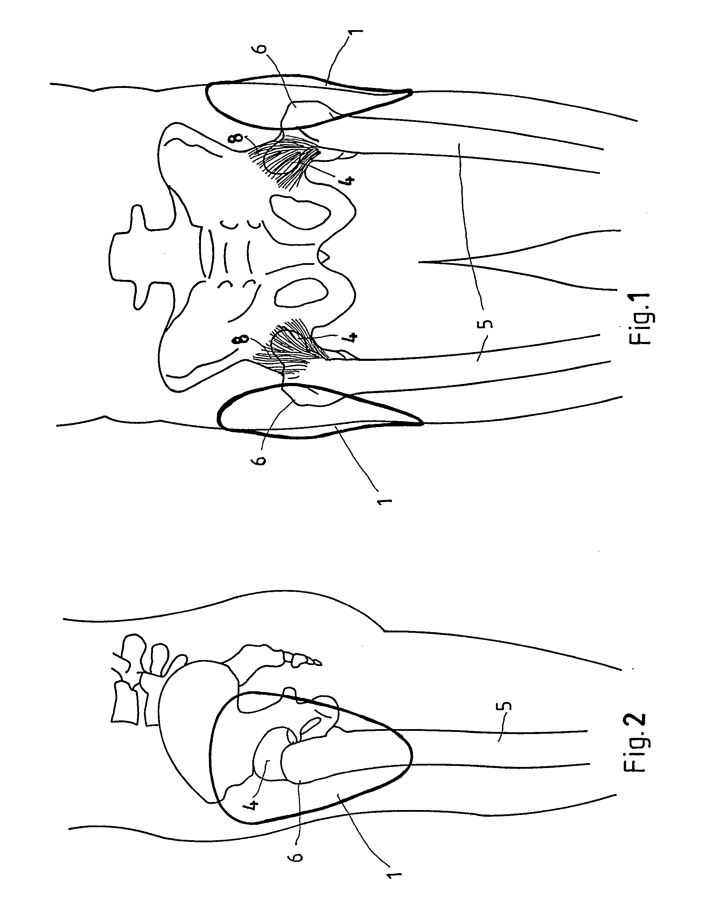

Die Fig. 1 und 2 zeigen den Beckenbereich des menschlichen Körpers. Jeweils in der Hüftgelenkkapsel 8 sitzt der Kopf 4 und der Hals des Oberschenkelknochens 5. Besonders fallgefährdet ist der grosse Rollhügel oder Trochanter major 6, der aus der Hüftgelenkkapsel 8 herausragt. Dieser Trochanter major 6 wird durch je einen Stossneutralisator 1 in der dargestellten Weise geschützt. Die Stossneutralisatoren können für die rechte und die linke Körperseite gleich oder paarig ausgebildet sein. Die Stossneutralisatoren werden so angelegt, dass jeweils der Bereich grösster Dicke über dem Trochanter major 6 liegt. Der Stossneutralisator wird auf der Haut durch eine hautfreundliche Haftklebeschicht befestigt.

Innerhalb des Stossneutralisators 1 kann man eine oder mehrere Luftkammern vorsehen. Die Luftkammern können auch als Poren ausgebildet sein.

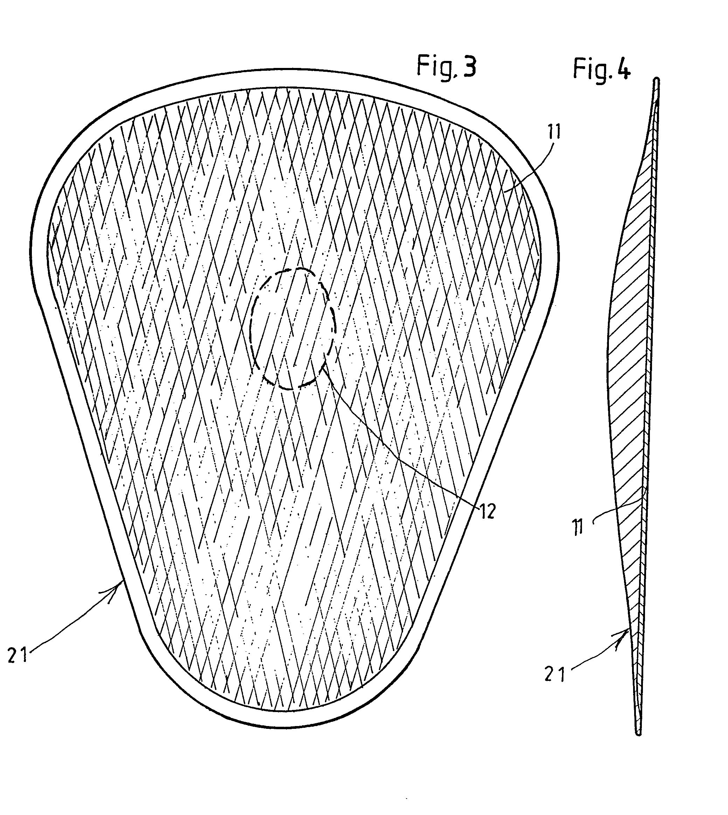

Eine Ausführungsform des Stossneutralisators ist in den Fig. 3 und 4 dargestellt. Der Stossneutralisator 21 hat einen Umriss in Ovalform. Der äussere Umriss kann selbstverständlich auch anders festgelegt sein. Im Querschnitt verläuft die äussere Begrenzungslinie auf der von dem menschlichen Körper abgewandten Seite glockenförmig oder hügelförmig, d. h. die Dickenabnahme ist zunächst gering; in einem mittleren Bereich nimmt die Dicke stark ab, und das Profil läuft am Rand flach aus. Im mittleren Bereich ist ein Auflagebereich 12 durch eine Umrisslinie gekennzeichnet, der dem grossen Rollhügel oder Trochanter major entspricht. Derselbe hat eine Ausdehnung von 6 bis 10 cm. Der Stossneutralisator hat eine Grundfläche von etwa 16 cm in der Breite und 20 cm in der Höhe. Diese Werte müssen an die jeweilige Körpergrösse angepasst werden. Die Auflagefläche der Unterseite des Stossneutralisators 21 ist im wesentlichen eben. Diese Auflagefläche ist mit einer Haftklebeschicht 11 versehen. Es handelt sich dabei um den gleichen Silikonkautschuk wie innerhalb der Grundschicht. Jedoch weist dieser Silikonkautschuk infolge eines geringeren Katalysatorzusatzes nur eine Teilvernetzung auf. Somit ist die Haftklebeschicht einerseits mit dem Grundkörper integral chemisch verbunden und hat andererseits fluidähnliche Eigenschaften. Sie ist ein zähflüssiges Fluid. Infolgedessen legt sie sich an die Haut des Trägers fest an. Vor allem werden die Poren der Haut ausgefüllt, so dass der Stossneutralisator dicht und klebend anliegt und damit eine hohe Schutzwirkung entfaltet.

Die Haftklebeschicht hat eine Dicke von einigen Millimetern, vorzugsweise zwischen 3 und 6 mm. Wie man der Schnittdarstellung der Fig. 4 entnimmt, hat der Stossneutralisator 21 eine flache, wannenförmige Ausnehmung, die durch die Haftklebeschicht 11 ausgefüllt ist. Die Gesamtdicke des Stossneutralisators beträgt 20 bis 50 mm, vorzugsweise 20 bis 40 mm. Dadurch wird unmittelbar über dem Trochanter major die grösste Dämpfungswirkung erzielt. Die dünneren äusseren Bereiche tragen zur Dämpfung dadurch bei, dass sie ein seitliches Ausweichen des Werkstoffes des Stossneutralisators verhindern.

Der als Klebstoff dienende untervernetzte Silikonkautschuk ist hautfreundlich. Der Stossneutralisator nach den Fig. 3 und 4 ist im wesentlichen symmetrisch und kann sowohl rechts als auch links getragen werden.

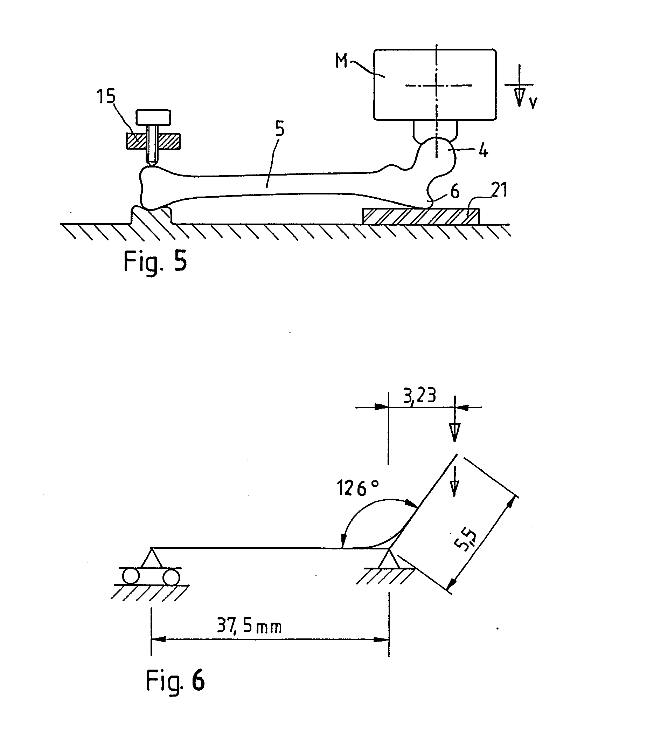

Die Wirkung des Stossneutralisators lässt sich durch eine linearisierte Theorie rechnerisch abschätzen und durch Versuche nachprüfen. Die Fig. 5 und 6 zeigen die entsprechenden Anordnungen und Grössenansätze. Nach Fig. 6 ist der Oberschenkelknochen 5 am Unterende in einer Spannvorrichtung 15 fest eingespannt. Der Trochanter major 6 liegt auf einem schematisiert dargestellten Schutzpolster 21 auf. Der Kopf 4 des Oberschenkelhalses wird durch eine Masse M belastet, die das reduzierte Körpergewicht darstellt. Das reduzierte Körpergewicht ist das mittlere Gewicht, das bei einem Fall wirksam ist. Das mittlere Körpergewicht liegt nach anerkannten Erfahrungswerten zwischen einem Drittel und der Hälfte des tatsächlichen Körpergewichtes. Denn der Sturz eines Menschen ist nicht ein freier Fall, sondern ein Kipp- oder Knickvorgang. Ausserdem ist der Körper des Menschen nicht starr, sondern durch Muskeln und Bänder elastisch zusammengehalten. Diese reduzierte Masse kann mit einer Geschwindigkeit v bewegt werden, um dadurch einen Fall zu simulieren.

Fig. 6 zeigt die Abmessungen, die für die Festigkeitsberechnung wichtig sind. Diese Abmessungen sind für einen repräsentativen Oberschenkelknochen 5 eingetragen. Wenn man die Stossenergie jeweils mit und ohne Schutzpolster betrachtet, so ergibt sich das Verhältnis der maximalen Stosskräfte wie folgt:

$$\frac{P_m}{P_o} \approx \sqrt{\frac{k_p}{k_F + k_p}}$$

mit $P_m$ als maximale Stosskraft mit Stossneutralisator, $P_o$ als maximale Stosskraft ohne Stossneutralisator, $k_p$ als Steifigkeit des Stossneutralisators und $k_F$ als Steifigkeit des Oberschenkelknochens einschliesslich des Hüftgelenkknorpels und der umgebenden Haut. Wenn man für diese Steifigkeiten Werte einsetzt, die sich aus modellmässigen Überlegungen ergeben, so ergibt sich

$$\frac{P_m}{P_o} = 0{,}30 \text{ bis } 0{,}45$$

Der untere Wert gilt für einen kleinen Kraftbereich von etwa 100 daN und der obere Wert für einen mittleren Kraftbereich von etwa 400 daN. Denn man muss die Rechnung kraftabhängig durchführen, weil die Verformungskennlinie des Stossneutralisators progressiv ist. Der obige Wert besagt, dass die Stosskraft beim Fallen auf den Trochanter major durch das Tragen des Stossneutralisators auf etwa 30 bis 45% der Stosskraft, die ohne Stossneutralisator wirksam würde, vermindert wird. Bei einer Stossneutralisatordicke zwischen 20 und 40 mm lässt sich somit eine sehr vorteilhafte Wirkung des Stossneutralisators erwarten. Es ist zu erwarten, dass durch Tragen des Stossneutralisators die Zahl der durch Fall verursachten hüftgelenksnahen Oberschenkelbrüche deutlich herabgesetzt werden kann.

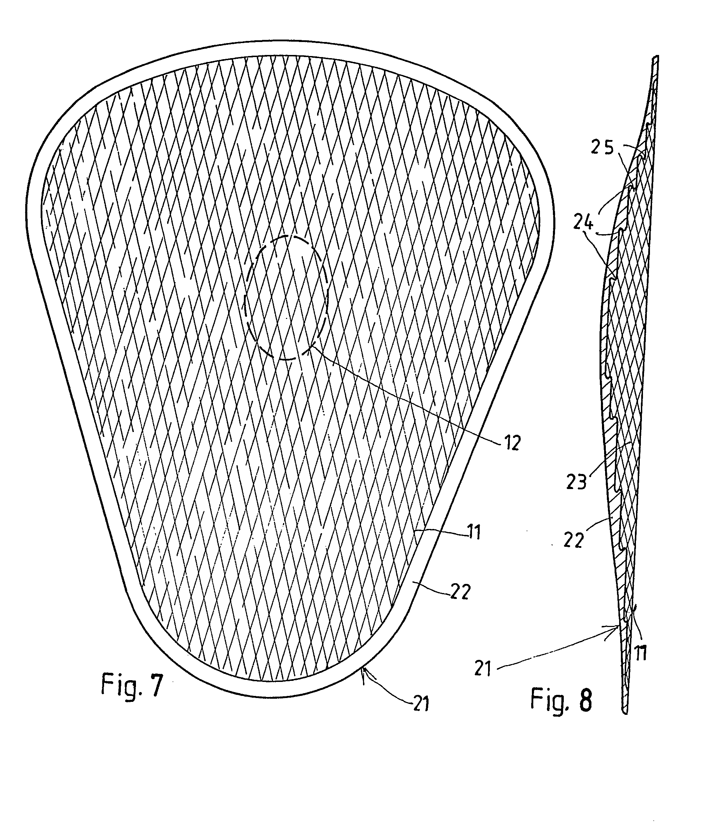

Der Stossneutralisator nach den Fig. 7 und 8 ist ähnlich wie der Stossneutralisator nach den Fig. 3 und 4 aufgebaut. Die Fluidschicht 23 aus dem untervernetzten Silikonkautschuk ist in Treppenstufen 24 aufgebaut, wobei die Umfangsflächen jeweils Hinterschneidungen 25 aufweisen. Eine schalenförmige Aussenschicht 22 umschliesst die Fluidschicht 23. Die Dicke der Fluidschicht 23 macht den Grossteil der Gesamtdicke des Stossneutralisators aus. Dieser Stossneutralisator hat eine hohe Absorptionswirkung, weil die Fluidschicht inkompressibel ist und bei einem Stoss oder Fall die gummielastische Aussenschicht 22 verdrängt. Dies bedeutet eine Speicherung der Stossenergie. Die Energie wird von der gummielastischen Aussenschicht 22 bei der Rückstellung verzögert und nur allmählich freigegeben. Man erzielt so eine hohe Absorber- oder Dämpfungswirkung.

**Patentansprüche**

1. Stossneutralisator (1, 21) zum Schutz des menschlichen Körpers mit einem Querschnitt, dessen äussere Begrenzungslinie auf der dem menschlichen Körper abgewandten Seite glockenförmig verläuft, und mit einem ovalförmigen Umriss sowie einem mehrschichtigen Aufbau, wobei die an der Haut anliegende Unterseite eine hautfreundliche Haftklebeschicht (1) aufweist, dadurch gekennzeichnet, dass eine an der Oberseite liegende Aussenschicht (22) aus einem gummielastischen Stoff besteht und dass innerhalb der schalenförmigen Aussenschicht (22) eine zähflüssige Fluidschicht (23) liegt, die durch chemische Bindungen in der Grenzschicht mit der Aussenschicht integral verbunden ist.

2. Stossneutralisator nach Anspruch 1, dadurch gekennzeichnet, dass die Aussenschicht (22) aus einem vernetzten Silikonkautschuk und die Fluidschicht (23) aus einem untervernetzten Silikonkautschuk besteht.

3. Stossneutralisator nach einem der Ansprüche 1 oder 2, dadurch gekennzeichnet, dass die Fluidschicht (23) an der Unterseite des Stossneutralisators (21) freiliegt und die Haftklebeschicht (11) bildet.

4. Stossneutralisator nach einem der Ansprüche 1 bis 3, dadurch gekennzeichnet, dass die Dikke der Fluidschicht (23) einige Millimeter bis 40 mm und die Gesamtdicke des Stossneutralisators 20 bis 50 mm beträgt.

5. Stossneutralisator nach Anspruch 4, dadurch gekennzeichnet, dass die Fluidschicht (23) am Umfang Treppenstufen (24) mit Hinterschneidungen (25) aufweist.

6. Stossneutralisator nach einem der Ansprüche 1 bis 5, dadurch gekennzeichnet, dass der Stossneutralisator (1) eine oder mehrere porenartige Luftkammern enthält.

7. Stossneutralisator nach einem der Ansprüche 1 bis 6, dadurch gekennzeichnet, dass zur Verwendung im Hüftbereich die Unterseite des Stossneutralisators im Bereich des Trochanter major eine kalottenförmige Ausnehmung aufweist.

**Claims**

1. An impact neutralizer (1, 21) for the protection of the human body having a cross-sectional area, the outer boundary line on the side opposite to the human body is bell-shaped, and having an oval outline and a multilayer construction, the bottom side overlaying the skin comprising a skin-friendly adhesive layer (11), characterized in that an outer layer (22) on the upper side consists of a rubber elastic substance, and that within the shell-shaped outer layer (22) a viscous fluid layer (23) is situated which is integral bonded to said outer layer by chemical bound in the interface.

2. An impact neutralizer according to Claim 1, characterized in that the outer layer (22) consists of a cross-linked silicone rubber and the fluid layer (23) of an only partially cross-linked silicone rubber.

3. An impact neutralizer according to Claim 1 or 2, characterized in that the fluid layer (23) is exposed on the bottom side of the impact neutralizer (21) and forms the adhesive layer (11).

4. An impact neutralizer according to Claims 1 to 3, characterized in that the thickness of the fluid layer (23) is up to 40 mm, and the total thickness of the impact neutralizer is from 20 to 50 mm.

5. An impact neutralizer according to Claim 4, characterized in that the fluid layer (23) has steps (22) with undercuts (25) at the periphery thereof.

6. An impact neutralizer according to any of the Claims 1 to 5, characterized in that the impact neutralizer (1) contains one or more pore-like air chambers.

7. An impact neutralizer according to any of the Claims 1 to 6, characterized in that for application in the area of the hip, the bottom side of the impact neutralizer has a concave recess for accommodating the greater trochanter of the hip joint.

**Revendications**

1. Dispositif de neutralisation de chocs (1, 21) pour la protection du corps humain, présentant une section transversale, dont la ligne limite extérieure est disposée en forme de cloche du côté opposé au corps humain, et un contour de forme ovale ainsi qu'une structure à plusieurs couches, la face inférieure en appui sur la peau présentant une couche auto-adhésive (11) compatible avec la peau, caractérisé en ce qu'une couche externe (22) située du côté supérieur est constituée d'une matière élastique caoutchouteuse et en ce que, à l'intérieur de la couche externe en forme de coque (22), se trouve une couche fluide visqueuse (23) qui est reliée de manière intégrale à la couche externe par des liaisons chimiques dans la couche limite.

2. Dispositif de neutralisation de chocs suivant la revendication 1, caractérisé en ce que la couche externe (22) est constituée d'un caoutchouc de silicone réticulé et la couche fluide (23) d'un caoutchouc de silicone sous-réticulé.

3. Dispositif de neutralisation de chocs suivant l'une des revendications 1 ou 2, caractérisé en ce que la couche fluide (23) est libérée sur la face inférieure du dispositif de neutralisation de chocs (21) et forme la couche auto-adhésive (11).

4. Dispositif de neutralisation de chocs suivant l'une des revendications 1 à 3, caractérisé en ce que l'épaisseur de la couche fluide (23) est de quelques millimètres à 40 mm et en ce que l'épaisseur totale du dispositif de neutralisation de chocs est de 20 à 50 mm.

5. Dispositif de neutralisation de chocs suivant la revendication 4, caractérisé en ce que la couche fluide (23) présente sur son pourtour des marches d'escalier (24) avec contre-dépouilles (25).

6. Dispositif de neutralisation de chocs suivant l'une des revendications 1 à 5, caractérisé en ce que le dispositif de neutralisation de chocs (1) contient une ou plusieurs chambres à air en forme de pores.

7. Dispositif de neutralisation de chocs suivant l'une des revendications 1 à 6, caractérisé en ce que, pour l'utilisation dans la zone de la hanche, la face inférieure du dispositif de neutralisation de chocs présente un évidement en forme de calotte dans la zone du grand trochanter.

Fig.1

Fig.2

Fig. 3

Fig. 4

11

12

21

11

21

Fig. 5

Fig. 6

Fig. 7

Fig. 8